# EUROPEAN PATENT SPECIFICATION

(11) **EP 1 469 888 B1**
(45) Date of publication and mention of the grant of the patent: **08.06.2016**
(21) Application number: 02789972.3
(22) Date of filing: 27.11.2002
(51) Int. Cl.: A61K 48/00

(54) **STEM CELL SCREENING AND TRANSPLANTATION THERAPY FOR HIV INFECTION**
STAMMZELLEN-SCREENING UND TRANSPLANTATIONSTHERAPIE BEI HIV-INFEKTION
CRIBLAGE DE CELLULES SOUCHES ET TRAITEMENT PAR TRANSPLANTATION POUR UNE INFECTION PAR LE VIRUS VIH

(30) Priority: 29.11.2001 US 998832
(43) Date of publication of application: 27.10.2004
(73) Proprietor: Stemcyte, Inc., Arcadia, CA 91006 (US)
(72) Inventor: CHOW, Robert, Arcadia, CA 91006 (US); RODGERSON, Denis, O., Malibu, CA 90265 (US); PUNZALAN, Rubio, R., Torrance, CA 90503 (US); PETZ, Lawrence, D., Tarzana, CA 91356 (US)
(74) Representative: Walton, Seán Malcolm
(86) International application number: PCT/US2002/038436
(87) International publication number: WO 2003/045335

(56) References cited:
- US-A- 6 153 431
- STEINBERGER ET AL.: 'Functional deletion of the CCR5 receptor by intracellular immunization produces cells that are refractory to CCR5-dependent HIV-1 infection and cell fusion' PROC. NATL. ACAD. SCI. USA vol. 97, no. 2, 18 January 2000, pages 805 - 810, XP000999142
- SHIOHARA ET AL.: 'Alterations of the cyclin-dependent kinase inhibitor p19 (INK4D) is rare in hematopoietic malignancies' LEUKEMIA vol. 10, no. 12, December 1996, pages 1897 - 1900, XP002967112
- LEE ET AL.: 'An intricate web: chemokine receptors, HIV-1 and hematopoiesis' STEM CELLS vol. 16, no. 2, 1998, pages 79 - 88, XP002955707
- BUHLER ET AL.: 'CCR5-intrabody mediated gene delivery provides protection from HIV infection' IMMUNITY AND ALTERNATIVE VECTORS page 422A, XP002984047 abstract #1772
- SMITH ET AL: "Contrasting genetic influence of CCR2 and CCR5 variants on HIV -1 infection and disease progression", SCIENCE, AMERICAN ASSOCIATION FOR THE ADVANCEMENT OF SCIENCE, US, vol. 277, 15 August 1997 (1997-08-15), pages 959-965, XP002099015, ISSN: 0036-8075, DOI: 10.1126/SCIENCE.277.5328.959
- SAMSON M ET AL: "RESISTANCE TO HIV-1 INFECTION IN CAUCASIAN INDIVIDUALS BEARING MUTANT ALLELES OF THE CCR-5 CHEMOKINE RECEPTOR GENE", NATURE, NATURE PUBLISHING GROUP, UNITED KINGDOM, vol. 382, no. 6593, 22 August 1996 (1996-08-22), pages 722-725, XP000198207, ISSN: 0028-0836, DOI: 10.1038/382722A0
- GLUCKMAN ELIANE ET AL: "Therapeutic use of cord blood stem cells", M-S (MEDECINE SCIENCES), vol. 11, no. 1, 1995, pages 28-34, ISSN: 0767-0974
- HOWS J M: "Status of umbilical cord blood transplantation in the year 2001", JOURNAL OF CLINICAL PATHOLOGY, BMJ PUBLISHING GROUP, GB, vol. 54, no. 6, 1 June 2001 (2001-06-01), pages 428-434, XP002983921, ISSN: 0021-9746, DOI: 10.1136/JCP.54.6.428
- ROSU-MYLES M ET AL: "Characterization of chemokine receptors expressed in primitive blood cells during human hematopoietic ontogeny", STEM CELLS (MIAMISBURG), vol. 18, no. 5, 2000, pages 374-381, ISSN: 1066-5099

## Description

### BACKGROUND OF THE INVENTION

Human immunodeficiency virus (HIV) infection is most commonly treated with agents that interfere with viral replication, such as small molecule protease inhibitors, nucleoside analogues, and non-nucleoside reverse transcriptase inhibitors. These antiviral therapies have been relatively effective for reducing viral loads and restoring immune function. However, these drugs exhibit numerous side effects, require prolonged treatment that often induces drug resistance, and do not result in complete eradication of the virus from the body. As a consequence, a great deal of current research focuses on developing therapies which either enhance the ability of the immune system to neutralize HIV or interfere with the ability of the virus to infect immune cells. In particular, these therapies exploit the growing body of evidence that certain gene polymorphisms are associated with reduced susceptibility and disease progression (*see,* Roger et al. FASEB, 12:625-632 (1998); O'Brien et al. Hospital Practice, July 15 (1998); Hogan et al. Ann. Intern. Med., 134:978-996 (2001)).

Many of these beneficial polymorphisms are variants of receptors and ligands for receptors that mediate HIV entry into immune cells. Although human immunodeficiency virus type-1 ("HIV-1") uses the T cell surface molecule CD4 as a primary receptor, successful viral entry into and infection of a cell has been found to require the presence of a second molecule, or "co-receptor" (*see,* Clapham and Weiss, Nature, 388:230-231 (1997)). Seven co-receptor molecules have been identified, each of which are members of, or related to, the family of chemokine receptors, which are G-protein coupled receptors having seven transmembrane domains. The chemokine receptor CCR5, which selectively binds RANTES, MIP-1alpha, and MIP-lbeta, serves as a coreceptor for macrophage tropic-strains of HIV, whereas the stromal derived factor 1 (SDF-1) chemokine receptor CXCR4 is a coreceptor for T cell-tropic HIV strains. CCR3, CCR2b, and CCR1 serve as coreceptors for other less common HIV strains.

The first HIV resistance gene to be characterized was a polymorphism of the primary HIV coreceptor CCR5 *(see,* Dean et al. Science, 273:1856-1862 (1996); Liu et al. Cell, 86: 367-377 (1996)). A 32 basepair deletion of the CCR5 receptor (CCR5 delta 32) causes a frameshift mutation and deletion of the last three transmembrane domains. Individuals homozygous for such a deletion remain uninfected despite multiple sexual exposures to HIV. However, those heterozygous for this deletion are susceptible to infection, although progression to AIDS may be slowed. Another beneficial polymorphism is a point mutation at residue 303 of the CCR5 (CCR5m303), which creates a stop codon and deletion of the last five transmembrane domains and the cytoplasmic tail. This mutation confers resistance to HIV infection when associated with the CCR5 delta 32 mutation *(see,* Quillent et al. Lancet, 351: 14-18 (1998)). A single amino acid substitution of CCR5, R89C also appears to confer resistance to HIV infection. (See, Tamasauskas et al., Blood, 97:3651-3654 (2001)). Polymorphisms (*e.g.,* CCR5P1, CCR5 59029A and 59353C) in the promoter region of these coreceptors are also associated with rapid progression of the disease (*see,* Ometto et al. J. Infectious Disease, 183:814-818 (2001); Martin et al. Infectious Disease, 282:1907-1911 (1998); Clegg et al. AIDS, 14:103-108 (2000)).

Variants of other less utilized HIV coreceptors also appear to influence disease progression. A conservative point mutation of the CCR2 receptor. CCR2-64I, permits expression of the receptor but nevertheless delays disease progression *(see,* Smith et al. Science, 277:959-965 (1997)).

Polymorphisms of genes encoding ligands for the HIV coreceptors CCR5 and CXCR4 influence disease progression, but not susceptibility. For example, homozygosity for a point mutation in the 3' untranslated region of a Stromal-derived Factor 1 alpha (SDF-1 alpha) delays disease progression in a recessive manner *(see,* Winkler et al. Science, 279:389-393 (1998)). It is hypothesized that the 3'A mutation upregulates the biosynthesis of SDF-1 alpha such that there is increased competition with HIV for CXCR4 receptors. A RANTES promoter polymorphism that increases RANTES expression is believed to function in a similar manner, but in this case by increasing competition with HIV for the CCR5 receptor *(see,* Liu et al. PNAS, 96:4581-4585 (1999)).

Finally, there is also evidence that HLA alleles influence HIV-1 disease progression. Animal studies demonstrate that resistance to murine AIDS maps to the H-2 complex, the mouse homologue of the HLA locus *(see,* Makino et al. J. Immunol., 144: 4347-4355 (1990)). The HLA complex contains three types of genes (class I, II, and III), all of which are involved in modulating the immune response. Class I (A, B, C, D, E, F,G) and class II (DM, DP, DQ, DR) molecules, commonly known as MHC genes, are both involved in antigen presentation to T cells. Class III HLA includes a variety of unrelated proteins, including the transporter for antigen processing (TAP), polypeptides of the proteasome, complement component factors (Bf, C2, C4), and tumor necrosis factors (TNF-alpha, TNF-beta).

Studies indicate that an individual's particular type of MHC class I and II molecules can influence disease progression. A study of pairs of HIV-1 infected hemophiliac brothers has demonstrated that sibling pairs sharing one or two HLA class II alleles exhibit similar rates of disease progression *(see,* Kroner et al. AIDS, 9:275-280 (1995)). A more recent study has found that HLA class I B*5701 is highly associated with restriction of viral replication in nonprogressors *(see,* Flores-Villanueva et al. PNAS, 98:5140-5145 (2001)). It is hypothesized that an enhanced ability of certain MHC proteins to associate with processed HIV-1 antigens allows certain individuals to mount a highly effective CD8 lymphocyte response against the virus.

Another polymorphism that influences HIV disease progression is IL10-5'A, a variant of the promoter region for interleukin-10 (IL-10). This polymorphism reduces IL10 production and is associated with rapid progression of AIDS in both homozygotes and heterozygotes (*see,* Shin et al. PNAS, 97:14467-72 (2000)). IL-10 is known to inhibit macrophage, T-lymphocyte, and HIV replication. Presumably, promoter mutations which increase IL-10 levels would slow progression of AIDS.

The discovery that certain polymorphisms confer resistance to HIV has led to the proposal of therapies which repopulate the immune system with cells more capable of resisting infection and/or more capable of neutralizing the virus. By preventing *de novo* infection of cells, such therapy can eliminate the need for prolonged treatment with inhibitors of viral replication. Furthermore, the specific nature of such therapies should reduce side effects.

WO 99/23253 and US Patent No. 6,153,431 describe vectors that can be used to express beneficial polymorphisms in existing lymphocytes or stem cells, suggesting the replacement of infected cells with transduced cells.

However, transducing circulating T lymphocytes with disease resistance polymorphisms is problematic, since these cells are so widely disseminated that is it is difficult to reach all target cells using current vector delivery systems. Furthermore, *in vitro* genetic engineering of stem cells and gene therapy with such cells can also be problematic. It is difficult to cultivate and transduce stem cells *in vitro.* Beneficial genes may not be expressed at sufficiently high levels to be effective, genes allowing infection by HIV may not be effectively "knocked out" using present methods, and transduction may affect subsequent differentiation into cells of the immune system. Finally, infusions of stem cells from donors, whether *in vitro* engineered or not, are preferably performed after matching of HLA phenotypes. Differences between the donor and the recipient can cause rejection of the transplant or even worse, the immune cells of the donor tissue may attack the tissues of the host (graft-versus-host disease). Current methods do not allow rapid and efficient identification of cells expressing both the desired disease resistance genes and HLA phenotype.

Thus there remains a need for a method for treating HIV infection that effectively renders immune cells refractory to HIV infection and/or enhances the ability of the immune system to neutralize the virus with a reduced risk of immunologic incompatibility. This invention fulfills this and other needs.

### BRIEF SUMMARY OF THE INVENTION

This invention relates to preventing or treating any disease arising from HIV infection, including AIDS and AIDS-related complex (ARC). The invention is as defined by the claims.

Other advantages, aspects, and embodiments of this invention will become apparent upon reading the following description.

### BRIEF DESCRIPTION OF THE DRAWINGS

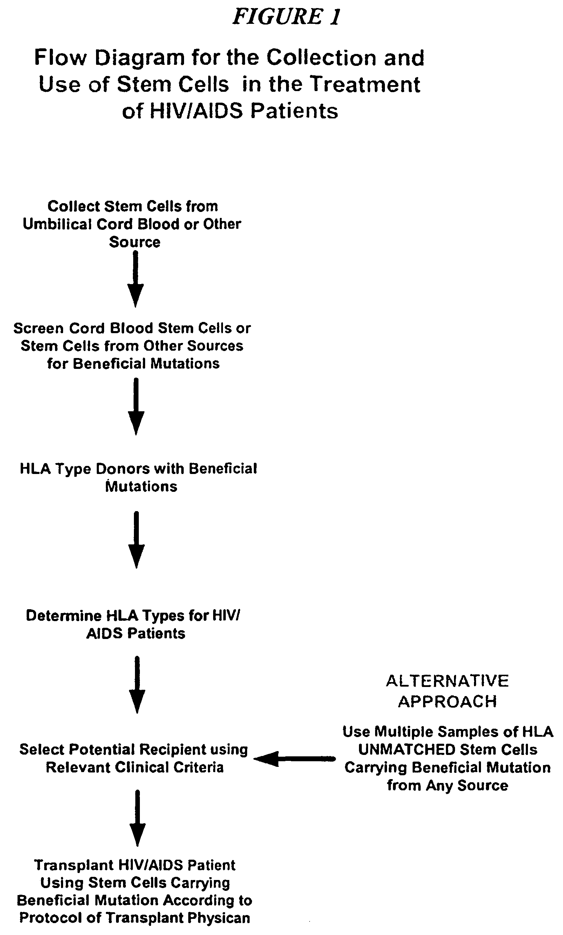

Figure 1 illustrates a flow diagram for one embodiment of the collection and use of stem cells in the treatment of HIV/AIDS patients.

### DETAILED DESCRIPTION

### I. Definitions

The term "beneficial gene" as described herein refers to any gene which provides increased resistance to a disease, any gene which slows or reduces the progression of a disease, or any gene which is beneficial to research. Beneficial gene can refer to a single allele of a gene or to the two alleles typically found in a diploid organisms. Genes beneficial to research includes those that could be used to produce mammalian models of various diseases.

The term "cells from a donor" refers to any population of cells that contains stem cells and is extractable from a donor. Typical sources of such cells include embryos, bone marrow, peripheral blood, umbilical cord blood, placental blood, adipose tissue, and any other tissue in which stem cells reside.

An "isolated population of human cells" refers to a population of cells that has been extracted from a donor.

The term "immune cell" as used herein refers to any cell which plays a role in the body's defense against pathogens. The primary immune cell targets of HIV are macrophages and T lymphocytes.

The term "polymorphism" as described herein refers to a variant of the sequence of a particular gene. This includes differences in genotypes ranging in size from a single nucleotide site to a large nucleotide sequence visible at a chromosomal level.

The term *"in vitro* expansion" refers to the cultivation of mammalian cells in the laboratory. Such cells can be extracted from a mammal and additional quantities of cells generated by cultivation in the appropriate environment. If possible, stable cell lines are established to allow for continued propagation of cells.

The term "stem cell" refers to any cells that have the ability to divide for indefinite periods of time and to give rise to specialized cells. Stem cells emanate from all germinal layers (ectoderm, mesoderm, and endoderm). Typical sources of stem cells include embryos, bone marrow, peripheral blood, umbilical cord blood, placental blood, and adipose tissue. Stem cells can be pluripotent, meaning that they are capable of generating most tissues on an organism. For example, pluripotent stem cells can give arise to cells of the skin, liver, blood, muscle, bone, *etc.* In contrast, multipotent or adult stem cells can only give arise to limited types of cells. For example, the hematopoietic stem cell can only give arise to cells of the lymphoid and myeloid lineages. Viable cells are cells that are alive and frequently are capable of growth and division. Those of skill are aware of methods to determine the viability of cells, for example, ability to exclude trypan blue dye.

The term "HIV" or "human immunodeficiency virus" refers to HIV-1, HIV-2, and any other strains of the virus which contribute to the development of AIDS or AIDS-related complex (ARC).

The term "HIV infection" as used herein refers to any of the spectrum of conditions associated with HIV infection, ranging from asymptomatic seropositivity, through AIDS-related complex (ARC), to acquired immunodeficiency syndrome (AIDS).

The term "acquired immunodeficiency syndrome" or "AIDS" as described herein refers to defects in cellular immunity associated with a infection with HIV, low CD4 positive T lymphocyte counts, and increased susceptibility to opportunistic infections and malignant neoplasms.

The term "HLA complex" as used herein refers to the collection of genes on a chromosome that encode MHC class I, class II, and class III molecules. The "MHC class I" molecules are glycoproteins which present antigens to T helper cells, whereas "MHC class II" molecules present antigens to cytotoxic T cells. "MHC class III" molecules are secreted proteins, such as proteins of the complement pathway and tumor necrosis factor (TNF).

The term "TNF" or "tumor necrosis factor" as used herein refers to the two related cytokines produced by macrophages (TNF-alpha) and some T cells (TNF-beta). These factors are cytotoxic to tumor cells and play a role in the inflammatory response.

The term "complement" as used herein refers to any of the group of serum proteins which form the membrane-attack complex, a complex which mediates cell lysis.

### II. Introduction

This disclosure relates to, *inter alia,* a method for preventing or treating any disease arising from HIV infection, including AIDS and AIDS-related complex (ARC). The method comprises screening a plurality of cells to identify stem cells with a beneficial gene and then transplanting the stem cells into a patient. In certain embodiments, potential donors are first screened for beneficial mutations and then stem cells are extracted from these donors for transplantation. The invention is as defined in the claims.

### III. Beneficial Genes of this Invention

In this invention, the beneficial gene renders immune cells resistant to HIV infection. This gene is a homozygous CCR5 delta 32 polymorphism.

### IV. Cell Populations to be Screened in the Method of this Invention

In one embodiment, the methods of this invention comprise screening a plurality of cells from donors to identify persons and cells with a beneficial gene. The population of cells to be screened should include stem cells., in particular human neonatal or fetal hematoepoietic stem cells derived from umbilical cord blood or placental blood.

Preferably, the cells to be screened are obtained from sources which allow for rapid and easy collection of a cells from a variety of unrelated individuals. Screening of cells from unrelated individuals provides the greatest chance of identifying cells with both the beneficial gene and a compatible HLA genotype.

### V. Screening Stem cell-rich Cell Populations of this Invention

Cells are typically screened to identify cells with homozygous CCR5 delta 32 polymorphism. In certain embodiments, the cells are also screened for a HLA genotype compatible with the patient. The samples used for screening may consist of cells taken directly from a donor, or from cell lines established from donor cells. The cells can be screened simultaneously for beneficial genes and HLA genotype, or screened sequentially. Those cells with a beneficial gene and an appropriate HLA genotype are then prepared for transplantation into a patient.

### A. Screening for Beneficial Genes

Cells are typically screened for beneficial genes using standard methods known to those of skill in the art for detection of particular nucleic acid sequences or proteins. In order to allow for rapid identification of therapeutic stem cell units expressing a beneficial gene, the methods are preferably ones which can be used in a high-throughput manner. Each cell sample from a donor may be screened for a variety of beneficial genes simultaneously. Alternatively, multiple samples are screened for presence of a particular beneficial gene.

In some embodiments, the cells are screened for beneficial genes using standard nucleic acid hybridization-based methods, including PCR based methods. Those of skill will be able to obtain nucleic acid sequences for determining whether beneficial genes are present. In a particularly preferred embodiment, the cells are screened using a modification of the high-throughput HLA-typing methods described in U.S. Pat. App. No. 09/747,391, filed December 20, 2000. Briefly, the method comprises: a) isolating template nucleic acid from the donor cells; b) amplifying the template nucleic acid; c) hybridizing the template nucleic acid with an immobilized array of capture oligo nucleotides, each having a known nucleic acid sequence of the beneficial genes being screened for; and d) determining the particular capture oligonucleotide to which the template nucleic acid hybridizes, thereby determining whether the cells have a beneficial gene.

Cells can also be screened using microarray technology. The DNA samples can be hybridized to an 96-well array, each target containing a nucleic acid sequence corresponding to that of a beneficial gene, *e*.*g*., a polymorphism of interest. Images of the microarrays are collected using a CCD camera and analyzed to identify target elements associated with fluorescent signal. These elements indicate the stem cell samples that express the beneficial gene.

In other embodiments, the cells are screened for beneficial genes using any standard immunological methods suitable for detecting the protein product of a beneficial gene, *i.e.,* Western blotting, standard immunoassays, and flow cytometry. A general overview of immunoassay technology can be found in Harlow & Lane, Antibodies: A Laboratory Manual (1988). The proteins expressed by beneficial genes of the invention can be detected and/or quantified using any of a number of well recognized immunological binding assays (*see, e.g.,* U.S. Patent Nos. 4,366,241; 4,376,110; 4,517,288; and 4,837,168). For a review of general immunoassays, see Methods in Cell Biology: Antibodies in Cell Biology, volume 37 (Asai, ed. 1993) and Basic and Clinical Immunology (Stites & Terr, eds., 7th ed. 1991). Immunological binding assays (or immunoassays) typically use an antibody that specifically binds to a protein or antigen of choice (in this case the protein expressed by the beneficial gene or an antigenic subsequence thereof). The antibody may be produced by any of a number of means well known to those of skill in the art.

### B. Screening for HLA type

The HLA genotype of the cells can be determined by any number of means known to those of skill in the art. Preferably, the HLA genotype is determined using the high-throughput HLA typing method described in U.S. Pat. App. No. 09/747,391, filed December 20, 2000. Briefly, the method comprises: (a) isolating template nucleic acid from the cells; (b) amplifying the template nucleic acid to generate sufficient product for each allele of at least one gene locus to be determined; (c) hybridizing the template nucleic acid with an immobilized array of capture oligonucleotides, each having a known nucleic acid sequence of an HLA allele; and (d) determining the particular capture oligonucleotide to which the template nucleic acid hybridizes, thereby determining the genotype of the subject.

Cells can also be screened using microarray technology. The DNA samples can be hybridized to an 96-well array, each target containing a nucleic acid sequence corresponding to that of an HLA allele. Images of the microarrays are collected using a CCD camera and analyzed to identify target elements associated with fluorescent signal. These elements indicate the HLA genotype of the stem cell samples.

Other standard methods include serological and cellular typing *(see,* Terasaki and McClelland, Nature, 204:998 (1964)), restriction fragment length polymorphism (RFLP) analysis, hybridization of PCR amplified products with sequence-specific oligonucleotide probes (PCR-SSO) to distinguish between HLA alleles *(see,* Tiercy et al., Blood Review, 4: 9-15 (1990)), sequence-specific primer amplification (PCR-SSP) *(see,* Olerup and Zetterquist Tissue Antigens, 39: 225-235 (1992)), and Single-Stranded Conformational Polymorphism (SSCP). Commercially available methods can also be used to determine HLA genotype.

### VI. Transplantation of Stem Cell-rich Cell Populations into Patients

In certain embodiments, the cells containing beneficial genes are transplanted without HLA typing. In other embodiments, the cells are HLA typed to ensure compatibility with the recipient.

The number of matches of HLA markers depends on the needs of the user and the source of the stem cells. Stem cells that have been isolated from cord blood can be used with four of six, five of six, or six or six marker matches.

In some immunocompromised patients, graft versus host response can be attenuated and stem cells that are not perfectly matched can be used.

After screening, cells expressing the desired beneficial gene and appropriate HLA genotype are selected and prepared for transplantation. If desired, the therapeutic stem cell units are expanded *ex vivo* (*in vitro*) using standard methods used to culture stem cells and maintain stable cell lines. Alternatively, these cells can be expanded *in vivo.* These cells can be used for future transplantation procedures. In certain embodiments the stem cell-rich cell populations are further enriched for stem cells prior to transplantation. Methods to select for stem cells are well known in the art. For example, samples can be enriched by tagging cell-surface markers of undifferentiated hematopoietic stem cells (*e.g.,* CD34, CD59, Thyl, CD38 low, C-kit low, lin minus) with fluorescently labeled monoclonal antibodies and sorting via fluorescence-activated cell sorting (FACS). In other embodiments, a sample of the stem cell-rich population is transplanted without further enrichment.

Typically, the normal stem cell population (which ultimately produces the lymphocytes susceptible to viral replication) is eliminated or reduced prior to transplantation of the therapeutic stem cell units. Chemotherapy, radiation, or the techniques described in U.S. Pat. No. 6,217,867 are used to condition the bone marrow for appropriate engraftment of the transplant. Finally, therapeutic stem cell units expressing the beneficial gene are transplanted into the patient using standard methods.

In some embodiments, the isolated population of cells comprising stem cells also comprise a pharmaceutical carrier, preferably an aqueous carrier. A variety of aqueous carriers can be used, *e.g.,* buffered saline and the like. These solutions are sterile and generally free of undesirable matter. These compositions may be sterilized by conventional, well known sterilization techniques. The compositions may contain pharmaceutically acceptable auxiliary substances as required to approximate physiological conditions such as pH adjusting and buffering agents, toxicity adjusting agents and the like, for example, sodium acetate, sodium chloride, potassium chloride, calcium chloride, sodium lactate, albumin, anticoagulants such as CPD (citrate, phosphate, and dextrose), dextran, DMSO, combinations thereof, and the like. The concentration of active agent in these formulations can vary widely, and will be selected primarily based on fluid volumes, viscosities, body weight and the like in accordance with the particular mode of administration selected and the patient's needs.

In some instances stem cells from a specific donor will prove to be particularly useful in the practice of the invention. After the first donation, additional stem cells can be obtained with the donor's consent. Donor confidentiality will continue to be maintained if more than one donation is obtained.

### VII. Therapeutic Applications

The invention can be used to treat or prevent any disease or condition that arises from HIV infection, such as AIDS and ARC. It should be recognized that this invention can easily be practiced in conjunction with existing antiviral therapies to effectively treat or prevent disease.

The progression of HIV, disease can influence the selection of stem cells with beneficial diseases for transplant. Briefly, early in infection HIV, isolates are predominantly moncytotropic (M-tropic), that is their host range is generally limited to PBLs and cells of the monocyte/macrophage lineage. M-tropic HIV strains infect macrophages via CCR5. As infection progresses HIV isolates become increasingly T-cell-line tropic (TCL tropic), that is their host range is generally limited to T-cells. TCL-tropic HIV strains infect T-cells via CXCR4. Dual tropic HIV isolates are also known. Those of skill will recognize that the tropism of a particular HIV isolate can be determined by assaying the ability of an HIV strain to infect a cell line. That is, an M-tropic HIV strain will be able to infect a macrophage line, while a TCL tropic HIV strain will be able to infect a T-cell line. In addition, TCL tropic HIV, strains cause syncytia formation after infection and this can be detected by those of skill.

In some embodiments, stem cells with HIV resistant CCR5 polymorphisms are transplanted into patients infected with predominantly M-tropic HIV, viruses. HIV resistant CCR5 polymorphisms can also be used to treat patients with a mixture of M-tropic and TCL-tropic HIV strains, or dual tropic HIV strains.

Factors and events which form a theoretical basis for the embodiments of the invention are discussed herein. However, this discussion is not in any way to be considered as binding or limiting on the present invention. Those of skill in the art will understand that the various embodiments of the invention may be practiced regardless of the model used to describe the theoretical underpinnings of the invention.

### EXAMPLES

The following examples are offered to illustrate, but not to limit the claimed invention.

### Example 1:

This example illustrates one embodiment of a method of this invention (Figure 1). Stem cells are collected from umbilical cord blood or another suitable source and then screened for beneficial mutations. Alternatively, potential donors are screened for beneficial mutations using standard genotyping methods. Once stem cells and/or donors with beneficial mutations are identified, they are HLA-typed and matched with the HLA types of HIV/AIDS patients desiring treatment. Next, potential recipients are selected using relevant clinical criteria and the stem cells are transplanted according to standard stem cell transplantation protocols. In certain instances, stem cells are also transplanted into patients without HLA matching.

### Example 2:

This example illustrates the method of this invention when it is used to screen for the CCR5 delta 32 polymorphism. Immune cells from individuals homozygous for this deletion remain uninfected despite infection of the individual with HIV, presumably because the mutation prevents expression of the HIV coreceptor CCR5 at the cell surface.

Umbilical cord blood samples from unrelated infants were obtained from the Stemcyte umbilical blood cord bank. DNA was extracted from the enriched samples using the salt extraction method. The cells were first lysed and centrifuged. Then water was added and the sample was centrifuged again. The pellet was digested with Proteinase K. The DNA was then extracted by the addition of 6M Guanidine HCl and incubation at 70°C for several minutes. The sample was centrifuged again and the supernatant was precipitated with cold 95% ethanol. The pellet was then dried and resuspended in the appropriate buffer.

The DNA samples were sequenced and screened for the presence of a nucleic acid sequence corresponding to that of the CCR5 delta 32 polymorphism. Samples with cells containing homozygous or heterozygous polymorphisms were also HLA-typed using a commercial procedure.

Approximately five thousand umbilical cord samples were tested for presence of an HIV resistant, homozygous CCR5 mutation. Twenty-two samples with HIV resistant homozygous CCR5 delta 32 polymorphisms were identified. Approximately 500 samples with heterozygous CCR5 delta 32 polymorphisms were identified. The samples were also screened for HLA genotype and were cryopreserved.

Samples with the beneficial polymorphism and the closest HLA match to a patient are selected and transfused intravenously into patient for treatment of HIV infection.

## Claims

1. An isolated population of human cells comprising viable human hematopoietic stem cells from umbilical cord blood of a donor, wherein the stem cells comprise a beneficial gene of the donor that confers resistance to HIV infection, wherein the beneficial gene is a polymorphism of a gene that encodes a CCR5 receptor, the polymorphism being a homozygous CCR5 delta 32 polymorphism, wherein the isolated population of human cells further comprises a pharmaceutical carrier and an anticoagulant.

2. The isolated population of human cells according to claim 1 for use in a method of preventing or treating HIV infection in a patient by transplanting the cells into a patient.

3. The isolated population of human cells for use according to claim 2, wherein the stem cells further comprise an HLA genotype that is compatible with a recipient of the stem cells.

4. Use of hematopoietic stem cells from umbilical cord blood of a donor having a beneficial gene of the donor that confers resistance to HIV infection in the manufacture of a medicament for preventing or treating HIV infection in a patient by transplantation of the stem cells, wherein the manufacture comprises preparing the stem cells from a sample of umbilical cord blood of the donor, wherein the beneficial gene is a polymorphism of a gene that encodes a CCR5 receptor, the polymorphism being a homozygous CCR5 delta 32 polymorphism, and wherein the stem cells are not embryonic stem cells.

5. A method of identifying hematopoietic stem cells having a beneficial gene for use in preventing or treating HIV infection in a patient by transplantation of the stem cells, the method comprising screening a plurality of cells from a sample of placental or umbilical cord blood of a donor to identify stem cells having a beneficial gene of the donor, wherein the beneficial gene is a polymorphism of a gene that encodes a CCR5 receptor, the polymorphism being a homozygous CCR5 delta 32 polymorphism, wherein the stem cells are not embryonic stem cells.

6. The method of claim 5, further comprising *in vitro* expansion of said stem cells.

7. The method of claim 5, wherein said method further comprises identification of the HLA genotype or phenotype of said stem cells, optionally via a high-throughput method using allele-specific primers and HLA locus-specific capture oligonucleotides immobilized on a solid phase.

8. The method of claim 5, wherein said screening comprises identification of stem cells expressing the protein product of said beneficial gene, optionally wherein said protein product is detected or identified using an immunological assay.

9. The method of claim 5, wherein said screening comprises identification of stem cells with said beneficial gene, optionally wherein said beneficial gene is detected using a hybridization-based assay, a sequencing assay, or a functional assay.

10. The method of claim 5, further comprising treatment of said stem cells to express a non-native HLA protein or to inhibit expression of the native HLA protein.

## Patentansprüche

1. Isolierte Population menschlicher Zellen, umfassend lebensfähige menschliche hämatopoetische Stammzellen aus Nabelschnurblut eines Spenders, worin die Stammzellen ein vorteilhaftes Gen des Spenders umfassen, das Resistenz gegenüber einer HIV-Infektion verleiht, worin das vorteilhafte Gen ein Polymorphismus eines Gens ist, das für einen CCR5-Rezeptor kodiert, wobei der Polymorphismus ein homozygoter CCR5-Delta-32-Polymorphismus ist, worin die isolierte Population menschlicher Zellen ferner einen pharmazeutischen Träger und ein Antikoagulans umfasst.

2. Isolierte Population menschlicher Zellen nach Anspruch 1 zur Verwendung in einem Verfahren zur Vorbeugung oder Behandlung einer HIV-Infektion bei einem Patienten durch Transplantieren der Zellen in einen Patienten.

3. Isolierte Population menschlicher Zellen zur Verwendung nach Anspruch 2, worin die Stammzellen ferner einen HLA-Genotyp umfassen, der mit einem Rezipienten der Stammzellen kompatibel ist.

4. Verwendung hämatopoetischer Stammzellen aus Nabelschnurblut eines Spenders, die ein Resistenz gegenüber einer HIV-Infektion verleihendes vorteilhaftes Gen des Spenders aufweisen, zur Herstellung eines Medikaments zur Vorbeugung oder Behandlung einer HIV-Infektion bei einem Patienten durch Transplantieren der Stammzellen, worin die Herstellung das Herstellen der Stammzellen aus einer Probe von Nabelschnurblut des Spenders umfasst, worin das vorteilhafte Gen ein Polymorphismus eines Gens ist, das für einen CCR5-Rezeptor kodiert, wobei der Polymorphismus ein homozygoter CCR5-Delta-32-Polymorphismus ist, und worin die Stammzellen nicht embryonale Stammzellen sind.

5. Verfahren zur Identifizierung hämatopoetischer Stammzellen, die ein vorteilhaftes Gen aufweisen, zur Verwendung in der Vorbeugung oder Behandlung einer HIV-Infektion bei einem Patienten durch Transplantieren der Stammzellen, wobei das Verfahren das Screenen einer Vielzahl von Zellen aus einer Probe von Plazentaoder Nabelschnurblut eines Spenders umfasst, um Stammzellen zu identifizieren, die ein vorteilhaftes Gen des Spenders aufweisen, worin das vorteilhafte Gen ein Polymorphismus eines Gens ist, das für einen CCR5-Rezeptor kodiert, wobei der Polymorphismus ein homozygoter CCR5-Delta-32-Polymorphismus ist, worin die Stammzellen nicht embryonale Stammzellen sind.

6. Verfahren nach Anspruch 5, ferner umfassend In-vitro-Expansion der Stammzellen.

7. Verfahren nach Anspruch 5, worin das Verfahren ferner Identifizierung des HLA-Genotyps oder -Phänotyps der Stammzellen umfasst, gegebenenfalls durch ein Hochdurchsatzverfahren, das auf einer Festphase immobilisierte allelspezifische Primer und HLA-locusspezifische Einfangoligonucleotide einsetzt.

8. Verfahren nach Anspruch 5, worin das Screening Identifizierung von Stammzellen umfasst, die das Proteinprodukt des vorteilhaften Gens exprimieren, worin gegebenenfalls das Proteinprodukt unter Verwendung eines Immunassays detektiert oder identifiziert wird.

9. Verfahren nach Anspruch 5, worin das Screening Identifizierung von Stammzellen mit dem vorteilhaften Gen umfasst, worin das vorteilhafte Gen gegebenenfalls unter Verwendung eines hybridisierungsbasierten Assays, eines Sequenzierungsassays oder eines funktionellen Assays detektiert wird.

10. Verfahren nach Anspruch 5, ferner umfassend eine Behandlung der Stammzellen, so dass diese ein nicht natives HLA-Protein exprimieren oder Expression des nativen HLA-Proteins hemmen.

## Revendications

1. Population isolée de cellules humaines comportant des cellules souches humaines hématopoïétique viables de sang de corde ombilicale d'un donneur, les cellules souches comprenant un gène bénéfique du donneur qui confère une résistance à l'infection VIH, le gène bénéfique étant un polymorphisme d'un gène qui encode un récepteur CCR5, le polymorphisme étant un polymorphisme homozygote CCR5 delta 32, la population isolée de cellules humaines comprenant en outre un porteur pharmaceutique et un anticoagulant.

2. Population isolée de cellules humaines selon la revendication 1 pour une utilisation dans un procédé de prévention ou de traitement d'une infection VIH dans un patient par transplantation des cellules dans un patient.

3. Population isolée de cellules humaines pour une utilisation selon la revendication 2, les cellules souches comprenant en outre un génotype HLA qui est compatible avec un destinataire des cellules souches.

4. Utilisation de cellules souches hématopoïétiques de sang de corde ombilicale d'un donneur ayant un gène bénéfique du donneur qui confère une résistance à l'infection VIH, dans la fabrication d'un médicament pour la prévention ou le traitement d'une infection VIH dans un patient par transplantation des cellules souches, la fabrication comprenant préparer les cellules souches d'un échantillon de sang de corde ombilicale du donneur, le gène bénéfique étant un polymorphisme d'un gène qui encode un récepteur CCR5, le polymorphisme étant un polymorphisme homozygote CCR5 delta 32, et les cellules souches n'étant pas de cellules souches embryonnaires.

5. Procédé d'identification de cellules souches hématopoïétiques ayant un gène bénéfique pour une utilisation dans la prévention ou le traitement d'une infection VIH dans un patient par transplantation des cellules souches, le procédé comprenant un criblage d'une pluralité de cellules d'un échantillon de sang de placenta ou de corde ombilicale d'un donneur pour identifier des cellules souches ayant un gène bénéfique du donneur, le gène bénéfique étant un polymorphisme d'un gène qui encode un récepteur CCR5, le polymorphisme étant un polymorphisme homozygote CCR5 delta 32, et les cellules souches n'étant pas de cellules souches embryonnaires.

6. Procédé selon la revendication 5, comprenant en outre une expansion in vitro des cellules souches.

7. Procédé selon la revendication 5, le procédé comprenant en outre une identification du génotype ou phénotype HLA des cellules souches, en option par un procédé à haut débit utilisant des amorces spécifiques de l'allèle et des oligonucléotides de captation spécifique de locus HLA immobilisés sur une phase solide.

8. Procédé selon la revendication 5, le criblage comprenant une identification de cellules souches exprimant le produit de protéine dudit gène bénéfique, en option le produit de protéine étant détecté ou identifié en utilisant un test immunologique.

9. Procédé selon la revendication 5, le criblage comprenant une identification de cellules souches avec le gène bénéfique, en option le gène bénéfique étant détecté en utilisant un test à base d'hybridation, un test de séquencement ou un test fonctionnel.

10. Procédé selon la revendication 5, comprenant en outre un traitement des cellules souches pour exprimer une protéine non native HLA ou pour inhiber l'expression de la protéine native HLA.
